# DEMANDE DE BREVET EUROPEEN

(11) **EP 0 729 764 A1**
(43) Date de publication de la demande: **04.09.1996**
(21) Numéro de dépôt: 96830083.0
(22) Date de dépôt: 26.02.1996
(51) Int. Cl.: A61M 15/00

(54) **Conditionnement pour substances à pulvériser**

(30) Priorité: 02.03.1995 IT FI009541
(71) Demandeur: Magni, Giuseppina, 40129 Bologna (IT)
(72) Inventeur: Magni, Giuseppina, 40129 Bologna (IT)
(74) Mandataire: Martini, Lazzaro

(57) **Abrégé**

Conditionnement pour substances liquides, solides, en poudre ou en grains, destinées à la pulvérisation caractérisée en ce qu'elle est constituée d'un récipient (2) associable à un appareil pour la pulvérisation desdites substances, lequel est pourvu d'un élément de fermeture étanche (3) situé au-dessus (3), que l'on peut emporter et/ou perforer, et pourvu d'une appendice (30) pour l'emporter manuellement: le produit à pulvériser (1) étant contenu dans ledit récipient (2) en quantité connue et prédeterminée.

## Description

La présente invention a pour objet un conditionnement pour substances à pulvériser.

Des appareils pour aérosol sont connus, spécialement aérosol-thérapie, comprenants des moyens pour l'émission d'ondes ultrasoniques lesquelles, opportunement véhiculées par un medium, comme par exemple de l'eau contenue dans une chambre de l'appareil située au-dessus desdits moyens, frappent le fond d'une petite cuve dans laquelle est contenu un liquide médicamenteux, lequel, grâce à un'action énergisante réalisée sur celui-ci par les ultra-sons, est pulvérisé et ainsi inhalé par le patien.

Sont aussi connus des appareils pour aérosol, spécialement aérosol thérapie, lesquels sont munis d'un compresseur d'air lequel alimente un flacon contenant la préparation et dans lequel se réalise la véritable pulvérisation de la substance par effet de l'éspansion à son intérieur de l'air débité par le compresseur.

Est aussi connue l'utilisation de pulvérisateurs de dimensions réduites pour aérosol-thérapie dans lesquels est prevue une chambre d'expansion pour un gas propulsif contenu dans une bombe mélangé à la préparation médicamenteuse, de manière que l'expansion du gas dans ladite chambre provoque la pulvérisation de la préparation.

Il est aussi connu que dans les pulvérisateurs à ultra-sons du type appelé "à sec" la préparation est placée directement en contact avec la source des ultra-sons, c'est-à-dire sans aucun moyen intermédiaire. Mais ce-ci est la cause, après un certain temps, de la formation d'incrustations et saleté qu'on pourra difficilement enlever de l'appareil, et ce-ci provoque une chute du caractère fonctinnel de l'appareil et représente un véhicule potentiel de contamination chimique des produits introduits chaque fois dans le pulvérisateur.

Dans tous ces pulvérisateurs, la substance qui faut pulvériser doit être préparée et donc introduite dans l'appareil par l'usager lui même. Ce-ci comporte des nobreux inconvénients, entre autres une certaine imprécision dans le dosage des substances due à l'inexpérience de l'usager, dont l'abilité à doser peut être réduite par des problèmes de vue ou de mobilité comme dans le cas des personnes agées, une réduite precision et fiabilité des moyens employés pour un tel but, comme compte-gouttes, mesures et seringues, l'hygiène insuffisante qui accompagne ce type d'opération, qui est réalisée dans la plupart des cas dans un milieu domestique, c'est-à-dire hygiéniquement inadaptée, et est aussi la cause d'une inapropriée prise de médicaments particulièrement actifs qui requièrent un dosage très attentif, et le fait qu'une même cuve est souvent utilisée plusieurs fois sans être correctement stérilisée, avec un grand risque d'infections bactériennes et virales spécialement si la cuve est utilisée par plusieurs personnes, comme il peut advenir dans un cabinet déstiné à l'aérosol-thérapie ou à l'intérieur d'une famille qui utilise le même appareil ou aussi avec des appareil lués.

Le but principale de la présente invention est celui d'éliminer les précités incovénients.

A ce resultat on est parvenus, conformément à l'invention, en adoptant l'idée de réaliser un conditionnement pour substances à pulvériser, lequel est constitué par un récipent associable à un appareil pulvérisateur, où à l'intérieur du récipient est palcée une quantité prédeterminé de substances à pulvériser: ledit récipient étant pourvu d'au moin un élément de fermeture étanche que l'on peut emporter et/ou perforable.

Les avantages qui dérivent de la présente invention consistent essentielement en ce qu'il est possible prédisposer dans chaque conditionnement ou cuve l'exacte dose du produit à pulvériser, à utiliser seulement quand il faut, selon des quantités prédéterminées en relation à la thérapie à suivre et à la nature du produit, en assurant toujours le maximum d'hygiène et facilité d'utilisation, tout ce-ci à un coût très bas; qu'au-moin un conditionnement singulier ou multiple conformément à l'invention peut être preparé soit sur echelle industrielle que dans les pharmacies.

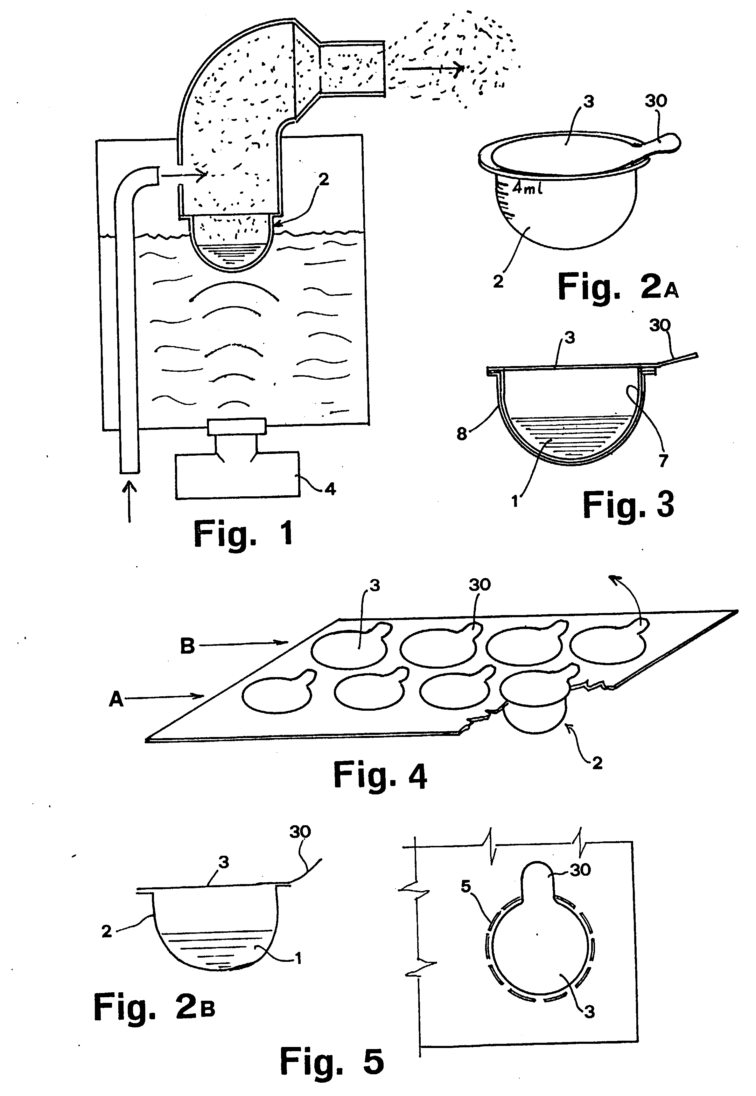

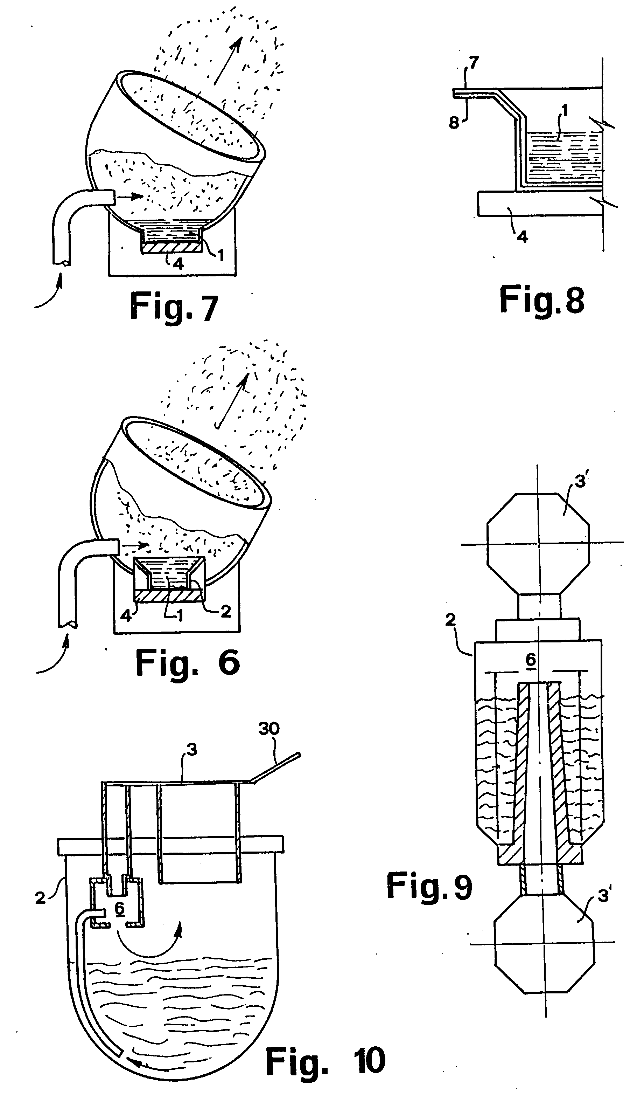

Ces avantages et caractéristiques de l'invention ainsi que d'autres seront plus et mieux compris de chaque homme du métier à la lumière de la description qui va suivre et à l'aide des dessins annexés donnés à titre d'exemplification pratique d'une forme concrète de réalisation, mais à ne pas considérer dans le sens limitatif, sur lesquels: la Fig. 1 représente, schématiquement, un appareil pulvérisateur à ultra-sons di type traditionel, dans lequel est logé un conditionnement conformément à l'invention, avec le produit pendant la pulvérisation; la Fig. 2A représente en vue perspective un conditionnement pour substances à pulvériser conformément à l'invention, selon une première forme de réalisation; la Fig. 2B représente schématiquement la vue en coupe verticale du conditionnement de la Fig. 2A; la Fig. 3 représente la vue en coupe verticale d'un conditionnement conformément à l'invention d'une deuxième forme de réalisation de l'invention; la Fig. 4 représente schématiquement une série de conditionnements pour substances à pulvériser conformément à l'invention placées sur un même véhicule du support; la Fig. 5 représente la vue en plan du détail d'un conditionnement du support de la Fig. 4; la Fig. 6 représente schématiquement un appareil pulvérisateur à ultra-sons du type appelé "à sec" avec un conditionnement conformément à l'invention; la Fig. 7 représente un appareil à ultra-sons en position d'utilisation traditionnelle, c'est-à-dire avec la préparation directement en contact avec la source des ultra-sons; la Fig. 8 représente la vue partielle en coupe verticale du conditionnement de la Fig. 6 conformément à une forme préférentielle de réalisation; la Fig. 9 représente schématiquement un conditionnement conformément à l'invention selon une autre forme de réalisation; la Fig. 10 représente schématiquement un conditionnement conformément à l'invention , où est prevu un seul élément de fermeture à couverture de deux sections de communication avec l'extérieur.

Réduit à sa structure essentielle et en reférence aux figures 1-8 des dessins annexés, un conditionnement pour substances liquides, solides, en poudre ou en grains, déstinées à la pulvérisation, particulièrement pour aérosol-thérapie, conformément à l'invention est constitué d'un récipient (2) associable à un appareil pour la pulvérisation desdites substances, lequel est pourvu d'un élément situé au-dessus (3) fermé étanche, que l'on peut emporter et/ou perforable, et pourvu d'une appendice (30) qui permet l'enlevement manuel: le produit à pulvériser (1) étant contenu dans ledit récipient (2) en quantité connue et prédeterminée. La fermeture de l'élément (3) au récipient (2) est réalisable selon n'importe laquelle technique apte, comme par exemple thermosoudure, assemblage avec des adhésifs ou solvants, utilisation d'ultra-sons ou fusion locale des matériaux qu'il faut assembler. Ledit récipient (2) est réalisable par moulage avec façonnage de la pièce sous-vide ou avec n'importe quel autre technique apte au but, comme par exemple injection ou soufflage du matériel de moulage dans des correspondantes moules ou impreintes.

Conformément à l'invention, ledit récipient (2) est avantageusement pourvu d'une échelle graduée pour simplifier l'emploi dans le cas où le produit (1) soit en forme solide ou en poudre ou en grains et requière l'adjuvant d'eau ou autres solvants et/ou intégrateur en quantité prédeterminée, avant l'usage, pour un dosage exacte des solvants en relation par rapport à la quantité et à la nature du produit (1).

Avantageusement, ledit élément (3) de fermeture est d'une épaisseur réduite, en guise de pellicule, en matériel plastique artificiel ou en complexe métal-plastique ou plastique-plastique.

Avantageusement, conformément à l'invention et en reférence aux Figg. 3 et 8 des dessins annexés, ledit récipient (2) est du type feuilleté, où la couche (7) intérieure est en matériel bio-compatible ou de toutes le façons inerte par rapport au produit (1) contenu à l'intérieur (par exemple polyéthylène) et celui estérieur (8) est par exemple en PVC rigide ou PET, pour être particulièrement apte à constituer élément de soutien structurale et de durcissement du récipient (2) et d'interface avec la source d'énergie de l'appareil sans subir déformations. Il en va de même pour l'élément de fermeture (3).

Il va de soi que plusieurs couches intermédiaires peuvent ê prvue entre les couche intérieur (7) et exetérieur (8).

Conformément à l'invention, avantageusement, plusieurs conditionnements sont obtenibles d'un même support sur lequel il y a des lignes pré-gravées (5), pour permettre l'assemblage de plusieurs unité séparables du support en exerçant une legère traction sur les lignes correspondantes de pré-gravure(5) et qui contiennent le même produit à pulvériser ou produits differents (A,B) (v. Figg. 4 et 5). Ce-ci se revèle particulièrement utile, par exemple, si l'on veut prédisposer dans un même conditionnement commercial plusieurs récipients (A) destinés à un anti-inflammatoire et plusieurs récipients (B) correspondants destinés à un substance mucolitique à inahler en succession pour en améliorer l'absorption au niveau des muqueuses.

Conformément à l'invention, avantageusement le fond dudit récipient (2) est concave, au autrement plat de manière à permettre l'appui directement sur la source d'énergie de l'appareil pulvérisateur, comme dans le cas des appareils à ultra-sons appelés "à sec" (v. Figg. 6, 7 et 8): la superficie extérne dudit récipient (2) étant avantageusement en matériel à base de silicone pour permettre la transmission d'énergie de la source (4) au produit (1) à pulvériser. Pour augmenter ultérieurement le rendement de la transmission énergetique de la source (4) au produit (1) il est aussi possible interposer un couche de gel ou de graisse entre le fond du récipient (2) et la surface active de la source (4), selon une méthodologie opérationelle déjà connue dans le secteur des techniques d'ecographie ultrasonique. Le gel ou la graisse destinés à une telle application sont en soi déjà connus par les hommes du métier et peuvent être fournis en tubes à presser associés au conditionnement commercial des récipients ou séparément.

Avantageusement, selon une autre forme de réalisation et en reférence à la Fig. 9 des dessins annexés, l'élément de fermeture du récipient (2) fait partie (3') de ce dernier et est détachable du même par simple torsion et coup sec.

En outre, avantageusemnt en reférence aux Figures 9 et 10 des dessins annexés, ledit récipient (2) est pourvu d'une bouche d'attache pour son reliement à un compresseur à air, comme aussi à un autre type de source de gas comprimé, par exemple oxigène, et d'une chambre (6) d'expansion du gas qui est prevue en aval de ladite bouche, et d'une sortie pour le produit pulvérisé: ladite sortie étant assemblable à des éventuels éléments de raccord et/ou accessoires de l'appareil pulvérisateur.

Avantageusement, conformément à l'invention, l'élément de fermeture du récipient peut être ouvrable de façon partielle, de manière à permettre l'utilisation de l'appareil en position incliné sans que le produit à pulvériser sorte du récipient.

Ce-ci est particulièrement utile dans le cas de la administration du produit pulvérisé à des patients allongés et aux enfants pendant le sommeil.

Il va de soi que le produit contenu dans ledit récipient (2) peut être non seulement pour aérosol-therapie, mais aussi pour d'autres et differentes applications, comme par exemple en dermatologie ou en cosmetique ou en agricolture ou dans l'industrie, selon des modalités en soi déjà connues aux hommes du métier.

Il va de soi, en outre, que l'appareil pulvérisateur au-quel est associable le conditionnement présent peut être aussi d'un type différent de ces surmentionnés.

## Revendications

1. Conditionnement pour substances liquides, solides, en poudre ou en grains, déstinées à la pulvérisation caractérisé en ce que est constitué d'un récipient (2) associable à un appareil pour la pulvérisation desdites substances, lequel est pourvu d'un élément situé au-dessus (3) fermé étanche, que l'on peut emporter et/ou perforable, et pourvu d'une appendice (30) qui en permet l'enlevement manuel: le produit à pulvériser (1) étant contenu dans ledit récipient (2) en quantité connue et prédeterminée.

2. Conditionnemen selon la revendication 1 caractérisé en ce que ledit élément (3) est pourvu d'une appendice (30) pour en permemttre l'enlevement manuelle.

3. Conditionnement selon la revendication 1 caractérisé en ce que ledit crécipient (2) est pourvu d'une echelle graduée.

4. Conditionnement selon la revendication 1 caractérisé en ce que ledit élément (3) de fermeture est d'une épaisseur réduite, en matériel plastique artificiel ou en complexe métal-plastique ou plastique-plastique.

5. Conditionnement selon la revendication 1 caractérisé en ce que ledit récipient (2) est du type feuilleté, où la couche (7) intérieure est en matériel bio-compatible ou inerte par rapport au produit (1) contenu à l'intérieur et la couche extérne est en matériel plastique rigide.

6. Conditionnement selon la revendication 1 caractérisé en ce qu'il est associé à des autres conditionnements du même type sur un même support ayant des lignes pré-gravées (5), pour permettre l'assemblage de plusieurs unités séparables du support et contiennant éventuellemnt le même produit à pulvériser ou produits differents (A,B).

7. Conditionnement selon la revendication 1 caractérisé en ce que le fond dudit récipient (2) est concave.

8. Conditionnement selon la revendication 1 caractérisé en ce que le fond dudit récipient (2) est plat.

9. Conditionnement selon les revendications 1 et 8 caractérisé en ce que la fond extérne dudit récipient (2) est en matériel à base de silicone.

10. Conditionnement selon la revendication 1 caractérisé en ce que l'élément de fermeture du récipient (2) fait partie (3') de ce dernier et est détachable du même par simple torsion et coup sec.

11. Conditionnement selon la revendication 1 caractérisé en ce que ledit récipient (2) est pourvu d'une bouche d'attache pour son reliement à un compresseur à air, comme aussi à un autre type de source de gas comprimé, et d'une chambre (6) d'expansion du gas qui est prevue en aval de ladite bouche, et d'une sortie pour le produit pulvérisé: ladite sortie étant assemblable à des éventuels éléments de raccord et/ou accessoires de l'appareil pulvérisateur.

12. Conditionnement selon la revendication 1 caractérisé en ce que l'élément de fermeture du récipient (2) est ouvrable seulement partiellement.
